Europäisches Patentamt

European Patent Office

Office européen des brevets

(11) Publication number: **0 518 561 A1**

# EUROPEAN PATENT APPLICATION

(21) Application number: 92305147.8

(22) Date of filing: 04.06.92

(51) Int. Cl.5: **A61M 5/30**

(30) Priority: **13.06.91 US 714892**

(43) Date of publication of application:
**16.12.92 Bulletin 92/51**

(84) Designated Contracting States:
**AT BE CH DE DK ES FR GB GR IT LI LU MC NL PT SE**

(71) Applicant: **BIOJECT INC**
**7620 Southwest Bridgeport Road**
**Porland, Oregon 97224(US)**

(72) Inventor: **McKinnon, Charles Neal, Jr.**
**7 Park Paseo**
**Laguna Niguel, CA 92677(US)**
Inventor: **Wilcox, Carl Evan**
**7620 Southwest Bridgeport Road**
**Portland, Oregon 97224(US)**
Inventor: **Nakagawa, Takaaki**
**16276 Southwest 104th Avenue**
**Tigard, Oregon 97224(US)**

(74) Representative: **Williams, Trevor John et al**
**J.A. KEMP & CO. 14 South Square Gray's Inn**
**London WC1R 5LX(GB)**

(54) Ampule for needleless hypodermic injection device.

(57) An ampule (12,50) for holding an injectant for injection by a needleless injection device using compressed gas, has a nozzle section (24) with a nozzle length to nozzle diameter ratio (L/D) of approximately 2.0 to 5.0. A general conical throat section (22) is joined to the nozzle section (24), and has inner walls flaring outwardly away from the nozzle section. A first transition section joined to the throat section (22) has inner walls curving convexly away from each other. A second transition section joined directly to the first transition section has concavely curving inner walls leading into the ampule body (30,50). The conical throat opening is joined to the nozzle opening by a convex radius (20; R7) approximately equal to ten times the diameter (D) of the nozzle opening.

FIG.2.

## Background of the Invention

The field of the present invention is ampules for use with hypodermic injection devices. Various ampules for use with needleless hypodermic injection devices have been proposed. See for example, EPO Application No. 90311965.9. Since the ampules are generally disposable, it is advantageous if they can be easily manufactured at low cost. In addition, the ampules are preferably easily sterilized and filled with injectant. Transparent materials are preferred for manufacturing ampules, to allow inspection of ampule contents and volume prior to injection. Ampules must have sufficient structural strength to withstand injection pressures, as well as reaction forces exerted by the needleless injection device on the ampule during injection. The ampule chamber, throat and nozzle must be properly shaped to achieve good flow characteristics, such that the injectant is driven out of the ampule in a properly shaped jet which can penetrate the skin.

Accordingly, it is an object of the invention to provide an improved ampule for use with a needleless hypodermic injection device.

## Description of the Invention

The ampule preferably comprises a generally cylindrical body having ampule walls which form an injectant chamber. A throat extends and tapers from the injectant chamber to a nozzle at one end of the ampule. Cam flanges may be provided at the other end of the ampule for securing the ampule to an injection device. Key tabs on the ampule adjacent and perpendicular to the flanges can be provided for disengaging a locking system on the injection device.

The ampule most desirably also has a generally cylindrical injectant chamber and a transition zone having a concave section adjoining the injectant chamber. A convex section adjoins the concave section and a conical section adjoins the convex section. A nozzle at the front end of the ampule adjoins the conical section to reduce pressure losses and lower turbulence.

The nozzle length to diameter (L/D) ratio ranges from about 2-5. The throat flare angle is advantageously about 10°.

## Brief Description of the Drawings

In the drawings wherein similar reference characters denote similar elements throughout the several views:

Figure 1 is an enlarged section view of an ampule;

Fig. 2 is a section view of a second preferred ampule embodiment;

Fig. 3 is a side elevation view thereof;

Fig. 4 is an enlarged view fragment of the nozzle of the ampule of Fig. 2;

Fig. 5 is a section view of a second preferred ampule embodiment;

Fig. 6 is a side elevation view thereof;

Fig. 7 is a section view fragment of tooling for molding ampule nozzles; and

Fig. 8 is an enlarged view fragment of the nozzle end of the ampule of Fig. 1.

## Detailed Description of the Preferred Embodiments

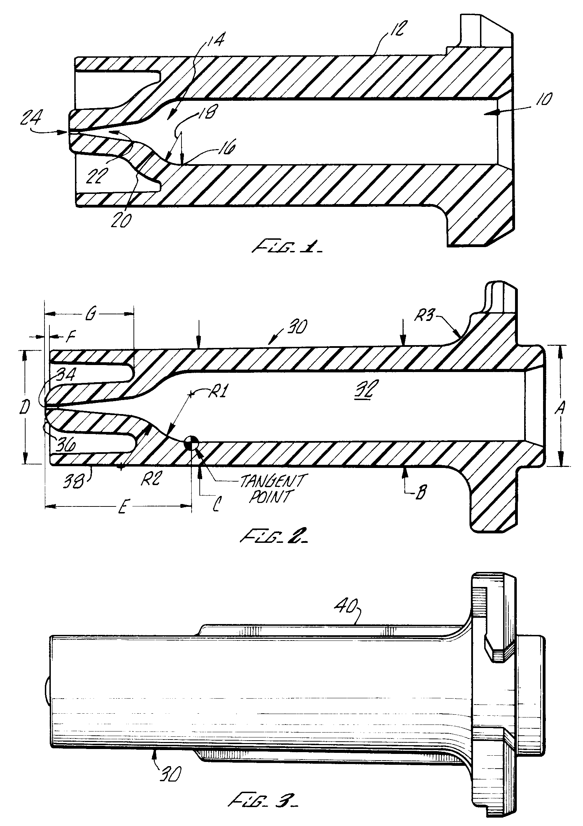

Referring to Figure 1, the ampule 12 has a generally flat rear surface 13. The ampule, which is preferably injection molded as a single part of a polycarbonate such as clear LEXAN HP2, has an injectant chamber 10, a transition zone 14 and a nozzle 24. A first or concave radius 18 begins at a tangent point 16. A second or convex radius 20 adjoins the first radius 18. A conical taper 22 extends from the nozzle 24 to the second radius 20. This provides a smooth transition zone 14 between injectant chamber and nozzle to avoid stress concentrations in the ampule 12 and provide good injection characteristics.

Fig. 8 shows preferred dimensions of the nozzle end of ampule 12, with R1 and R2 at .48 cm, dimension E at 1.10 cm in. angle alpha at 14 degrees and an injectant chamber inside diameter of .64 cm nominal.

The nozzle 24 extends a short distance before joining the conical taper 22. The nozzle diameter can vary, depending on application, e.g., intramuscular, subcutaneous, veterinary, etc.

Generally, with hand power syringe/needles, the interior pressures are limited and rarely or never exceed about 200 kpa. This limits the pressure producing forces on the ampule. Thus, the syringe can be thin-walled since stresses produced by the compressed injectant are low. On the other hand, with needleless compressed gas powered injection devices, the pressures generated within the ampules may be orders of magnitude greater, i.e., in the range of approximately 20,000-40,000 kPa. These higher pressures are necessary to generate a liquid jet with sufficient velocity to pierce the skin without a needle.

To achieve such a jet velocity that will pierce the skin at minimum pain levels, two basic jet characteristics should be provided:

1. The velocity profile across the jet diameter should be as flat as possible (not parabolic as in highly viscous tubular flow). This ensures that maintain of the jet will be sufficient, not only to initially pierce the skin, but also to maintain control of depth of penetration.

2. Turbulence in the jet should be minimized to enhance axial momentum and additionally to minimize jet spreading due to turbulent transverse velocity components.

These characteristics can be achieved with proper nozzle design, in particular, the converging section between the larger cylindrical bore and the final cylindrical nozzle section. The converging transition should be without any rapid or step changes in cross-section. Long linearly tapered nozzles can provide characteristics 1 and 2 above. However, such designs have larger pressure losses, and are less practical for a disposable device since they use relatively large volumes of material. At the other extreme, a sharp edged hole in a perpendicular cylinder end can work as a nozzle but the resulting jet will generally not have characteristics 1 or 2 above. The preferred compromise, considering pressure losses versus characteristics 1 and 2 above and material requirements, is to have a short nozzle using connecting radii. This preferred design yields the least turbulence for given length.

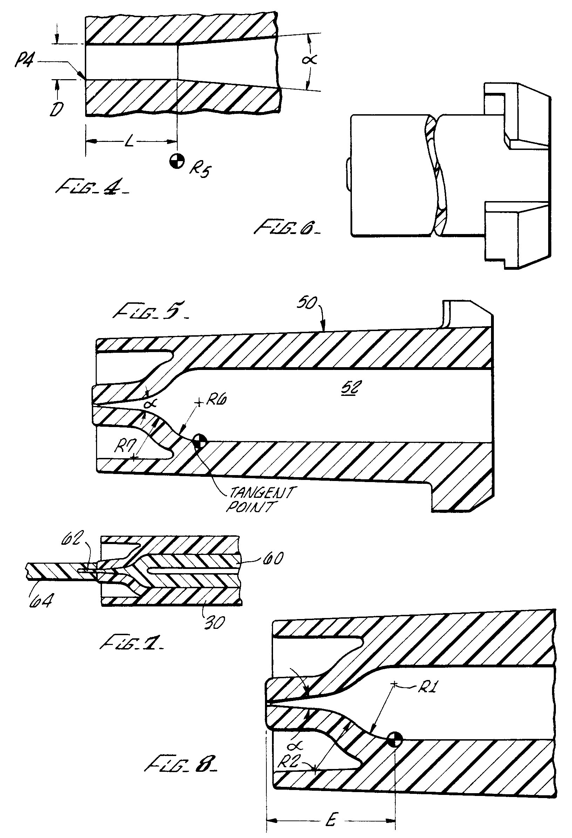

The ampules shown in the Figures are designed to withstand the relatively higher injectant pressures generated during the injection sequence. One critical area in the ampule design is the Luer tip end. Fig. 4 depicts the nozzle and throat of the ampule shown in Fig. 1, as well as the ampule 30 shown in Fig. 2 and the ampule 50 shown in Fig. 5. Referring to Fig. 4, with a short nozzle length, the fluid cone angle alpha would conventionally be large, in the range of, for example, 20-30 degrees. In contrast, the standard Luer tip dimensions require a certain minimal axial length. The critical stress intensity occurs at the minimum section thickness, where the Luer taper transitions to the radius extending to the injectant chamber diameter. Thus, the fluid cone angle alpha must be reduced to keep stresses within safe limits. Resulting fluid cone angles (alpha) of 4-12 degrees are accordingly preferred, although these angles incur a small additional pressure drop.

Another stress concentration area of the ampules is at the rear or lug end. The ampules have three lugs which are used to attach the ampule to an injection device at the outer edge of the lugs. During injection, there is about a 90 Kilogram-force axial load divided among the three lugs, in addition to the internal radial pressure loading on the ampule. This axial load causes high stress intensities at the corners where the lugs attach to the cylindrical ampule body. Accordingly, the lugs preferably have a gradual change in cross-section and a relatively large radius (for example, .24 cm) joining the lugs to the ampule body, as shown at R3 in Fig. 2.

Since the ampules are preferably disposable, their cost is minimized by reducing the material required for each ampule. Sufficient material must nevertheless be provided to maintain stresses at acceptable levels. Ampule 30 shown in Fig. 2 illustrates an ampule similar to ampule 12 but manufactured using less material.

Referring still to Fig. 2, the preferred contour design of the transition from the injectant chamber 32 of the ampule 30 to the nozzle 34 is defined by the concave radius R1 and convex radius R2, preferably both .48 cm. The nozzle end 36 of the ampule 30 protrudes by dimension F, preferably .05 cm beyond the ends of the shroud 38. This protrusion facilitates secure engagement of the nozzle against the patient's skin. The shroud length indicated by dimension G is preferably .84 cm and the dimension E from the tangent point to the nozzle is preferably 1.27 cm. The base or lug end diameter A is preferably 1.07 cm. The wall thickness most desirably ranges from .21 cm near position B where the ampule outside diameter is 1.05 cm, to .19 cm at position C where the ampule outside diameter is 1.02 cm. With a total overall ampule length of 4.37 cm, the spacing between positions B and C is preferably 2.09 cm, with position B .94 cm from the lug end of the ampule. The injectant chamber diameter 32 nominally has a preferred diameter of .64 cm. The ampule is designed for manufacture by injection molding and has appropriate draft angles. The nozzle end of ampule 30 is configured to the standard male Luer fitting. The angle alpha is preferably 10 degrees. As shown in Fig. 3, the ampule 30 has a series of radially extending ridges 40 to facilitate gripping the ampule by hand.

Ampule 30 carries 0.5 ml volume of injectant. Ampule 50 shown in Fig. 5 is similar to the ampule 30 but is formed with an injectant chamber 52 having a larger nominal injectant chamber diameter (1.41 cm) and holding 1.0 ml of injectant. Radii R6 and R7 are .54 cm, alpha is 10 degrees, L is .05 cm, the tangent point is 3.10 cm from the lug end, and the overall length is 4.18 cm.

The nozzle section L/D ratio is another factor that helps maintain minimum jet spreading and transverse turbulent momentum. Ratios from 2 to 5 provide a good compromise to damp transverse velocity without incurring too high a nozzle pressure drop. An L/D ratio of 2.5 is most preferred. The nozzle diameter D ranges from approximately .010 to .064 cm. With an L/D ratio of 2.5, L ranges from .025 to .159 cm. The radius R5 in Fig. 4 is preferably 10xD.

P4 (Fig. 4) is preferably a sharp corner. A substantial radius at P4 will create a bell mouth design allowing the jet of injectant to possibly diverge as it exits the ampule during injection.

Fig. 7 shows manufacturing tooling for injection molding ampules, such as ampule 30. A core pin 60 has a fine wire tip 62 ground onto its leading end. The wire tip 62 extends into a matching hole in a pilot guide 64. The wire tip 62 allows for the injection molding of very small diameter orifice openings, facilitates sharp corners at P4 (Fig. 4), centers the nozzle opening and provides added rigidity to resist transverse flow of plastic during injection molding.

Thus, while several embodiments of the invention have been described, it will be obvious that many modifications may be made, without departing from the scope of the invention.

**Claims**

1.  An ampule comprising:
    a body having generally cylindrical walls defining an injectant chamber;
    a conical throat section having walls defining a throat opening with a larger diameter end and a smaller diameter end, said larger diameter end of said conical throat opening joining to said injectant chamber;
    a nozzle section joined to said throat section at the smaller diameter end of said throat opening, said nozzle section having a nozzle opening with a diameter D and a nozzle opening length L, with the ratio of L/D approximately in range of from 2 - 5.

2.  The ampule of claim 1 wherein the conical throat forms a solid angle in the range of approximately 4-12 degrees.

3.  The ampule of claim 1 wherein the larger diameter end of said throat opening is joined to said injectant chamber in said cylindrical body by a concave radius.

4.  The ampule of claim 1 wherein the smaller diameter end of said throat opening is joined to said nozzle opening by a convex radius.

5.  The ampule of claim 1 further comprising at least two lugs attached to the ampule for holding the ampule in an injection device.

6.  The ampule of claim 1 wherein said cylindrical body, conical throat section and nozzle section are injection molded as a single piece.

7.  The ampule of claim 1 further comprising a shroud substantially surrounding said nozzle section.

8.  The ampule of claim 7 wherein said nozzle section has a nozzle end that is coplanar with or protruding beyond the shroud.

9.  The ampule of claim 1 further comprising a Luer fitting around said nozzle section.

10. The ampule of claim 1 wherein said conical throat opening is joined to said nozzle opening by a convex radius approximately equal to 10D.

11. The ampule of claim 1 wherein the body walls have a thickness ranging from approximately .19 to .21 cm.

_FIG. 1_

_FIG. 2_

TANGENT POINT

_FIG. 3_

P4

D

L

*R5*

*Fig_4_*

*Fig_6_*

*Fig_5_*

50

52

+ R6

R7

α

TANGENT
POINT

62

60

64

30

*Fig_7_*

*Fig_8_*

+ R1

α

R2

E

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X,D | EP-A-0 427 457 (BIOJECT INC)<br>* column 16, line 6 - line 8 *<br>* column 16, line 30 - line 31 *<br>* column 18, line 37 - line 54 *<br>* figures 16,20,27,28 *<br>--- | 1-11 | A61M5/30 |
| X | EP-A-0 409 674 (PASTEUR MERIEUX SERMUS ET VACCINS)<br>* figure 1 *<br><br>------ | 1,2,4 | |

| | |
|---|---|
| | TECHNICAL FIELDS SEARCHED (Int. Cl.5 ) |
| | A61M |

The present search report has been drawn up for all claims

| Place of search | Date of completion of the search | Examiner |
|---|---|---|
| THE HAGUE | 17 SEPTEMBER 1992 | SEDY R. |

EPO FORM 1503 03.82 (P0401)